# EUROPEAN PATENT APPLICATION

(11) **EP 3 901 930 A1**
(43) Date of publication of application: **27.10.2021**
(21) Application number: 19900336.9
(22) Date of filing: 17.12.2019
(51) Int. Cl.: G08B 21/02, G08B 25/04, A61B 5/00, A61B 5/01

(54) **HEATSTROKE PREVENTION DEVICE, HEATSTROKE PREVENTION SYSTEM, HEATSTROKE PREVENTION METHOD, AND PROGRAM**

(30) Priority: 17.12.2018 JP 2018235668
(71) Applicant: CMIC Holdings Co. Ltd., Tokyo 105-0023 (JP)
(72) Inventor: SATO, Keizo, Sendai-shi, Miyagi 980-8577 (JP); NAGATOMI, Ryoichi, Sendai-shi, Miyagi 980-8577 (JP)
(74) Representative: Trinks, Ole
(86) International application number: PCT/JP2019/049325
(87) International publication number: WO 2020/129956

(57) **Abstract**

A heatstroke prevention device includes an auricle temperature sensor configured to measure an auricle temperature that is a temperature of an auricle of a biological body; a controller configured to determine a possibility of development of heatstroke of the biological body on the basis of the auricle temperature; and a notification means configured to give a notice that there is the possibility of the development of the heatstroke on the basis of a determination result of the controller. The controller includes a comparison unit configured to compare the measured auricle temperature and a preset threshold of the auricle temperature, and to determine whether there is the possibility of the development of the heatstroke on the basis of a comparison result, and an operation control unit configured to control operation of the notification means on the basis of a determination result of the comparison unit.

## Description

### TECHNICAL FIELD

The present invention relates to a heatstroke prevention device, a heatstroke prevention system, a heatstroke prevention method, and a program that predict the condition of a biological body and prevent heatstroke.

### BACKGROUND ART

There have been proposed a heatstroke prevention system and a heatstroke prevention device that prevent the heatstroke of a biological body. For example, Patent Literature 1 discloses a heatstroke prevention system that measures the ear cavity temperature, which is known to be close to the brain temperature closely related to the heatstroke, and that determines the possibility of development of the heatstroke on the basis of the measurement result. In this system, a temperature sensor formed in an earplug shape is worn by the biological sensor, and the ear cavity temperature is measured by the temperature sensor.

### CITATION LIST

### PATENT LITERATURE

Patent Literature 1: Japanese Unexamined Patent Application Publication JP 2012-179213 A

### SUMMARY OF THE INVENTION

### TECHNICAL PROBLEM

In many cases, a heatstroke is developed during exercise or working. Therefore, for preventing such a heatstroke, it is necessary to measure the ear cavity temperature during exercise or working. However, in the system described in Patent Literature 1, as the temperature sensor formed in an earplug shape is worn, a problem arises that the measurement of the ear cavity temperature during exercise or working is very bothersome to the biological body.

An object of the present invention, which has been made in view of the problem in the related art, is to provide a heatstroke prevention device, a heatstroke prevention system, a heatstroke prevention method, and a program that make it possible to prevent a heatstroke easily and surely while the biological body is not conscious of the measurement.

### SOLUTION TO PROBLEM

A heatstroke prevention device according to one embodiment of the present invention includes an auricle temperature sensor configured to measure an auricle temperature that is a temperature of an auricle of a biological body; a controller configured to determine a possibility of development of heatstroke of the biological body on the basis of the auricle temperature; and a notification means configured to give a notice that there is the possibility of the development of the heatstroke on the basis of a determination result of the controller.

The controller includes a comparison unit configured to compare the measured auricle temperature and a preset threshold of the auricle temperature for the heatstroke, and to determine whether there is the possibility of the development of the heatstroke on the basis of a comparison result, and an operation control unit configured to control operation of the notification means on the basis of a determination result of the comparison unit.

A heatstroke prevention device according to another embodiment of the present invention includes an auricle temperature sensor configured to measure an auricle temperature that is a temperature of an auricle of a biological body; a controller configured to determine a possibility of development of heatstroke of the biological body on the basis of the auricle temperature; and a communication circuitry configured to send a determination result of the controller to a server.

The controller includes a comparison unit configured to compare the measured auricle temperature and a preset threshold of the auricle temperature for the heatstroke, and to determine whether there is the possibility of the development of the heatstroke on the basis of a comparison result.

A heatstroke prevention system according to another embodiment of the present invention includes a wearable terminal; a server configured to perform communication with the wearable terminal; and a management device configured to perform communication with the server. The wearable terminal includes an auricle temperature sensor configured to measure an auricle temperature that is a temperature of an auricle of a biological body, and a notification means configured to give a notice that there is a possibility of development of heatstroke of the biological body.

The server includes an arithmetic device configured to compare the auricle temperature measured by the wearable terminal and a preset threshold of the auricle temperature, and to determine whether there is the possibility of the development of the heatstroke on the basis of a comparison result. The management device includes a storage unit configured to store a determination result of the server, and a display means configured to display the determination result.

A heatstroke prevention system according to another embodiment of the present invention includes a server configured to perform communication with a wearable terminal; and a management device configured to perform communication with the server. The server includes an arithmetic device configured to compare an auricle temperature and a preset threshold of the auricle temperature, and to determine whether there is a possibility of the development of heatstroke on the basis of a comparison result, the auricle temperature being a temperature of an auricle of a biological body and being measured by the wearable terminal. The management device includes a storage unit configured to store a determination result of the server, and a display means configured to display the determination result.

A heatstroke prevention method according to another embodiment of the present invention includes measuring an auricle temperature that is a temperature of an auricle of a biological body; comparing the measured auricle temperature and a preset threshold of the auricle temperature for heatstroke; and controlling operation of a notification means on the basis of a result of the comparing.

A program according to another embodiment of the present invention causes a computer of a wearable terminal to be used as a comparison unit and an operation control unit, the wearable terminal including an auricle temperature sensor configured to measure an auricle temperature that is a temperature of an auricle of a biological body, and a notification means configured to give a notice that there is a possibility of development of heatstroke, the comparison unit comparing the measured auricle temperature and a preset threshold of the auricle temperature for the heatstroke, and determining whether there is the possibility of the development of the heatstroke on the basis of a comparison result, the operation control unit controlling operation of the notification means on the basis of a determination result of the comparison unit.

### ADVANTAGEOUS EFFECTS OF THE INVENTION

As described above, in an embodiment of the present invention, the possibility of the development of aheatstroke of a biological body is determined on the basis of the measured auricle temperature. Thereby, it is possible to prevent a heatstroke easily and surely while the biological body is not conscious of the measurement.

### BRIEF DESCRIPTION OF DRAWINGS

- FIG. 1: is a block diagram showing an example of the configuration of a heatstroke prevention device according to Embodiment 1.
- FIG. 2: is a functional block diagram showing an example of the configuration of a controller in FIG. 1.
- FIG. 3: is a hardware configuration diagram showing an example of the configuration of the controller in FIG. 2.
- FIG. 4: is a hardware configuration diagram showing another example of the configuration of the controller in FIG. 2.
- FIG. 5: is a graph showing an example of the correlation between an ear cavity temperature and an auricle temperature from a resting time to an exercise ending time.
- FIG. 6: is a graph showing an example of the correlation between the ear cavity temperature and the auricle temperature during the resting time in FIG. 5.
- FIG. 7: is a graph showing an example of the correlation between the ear cavity temperature and the auricle temperature at the time of warm-up in FIG. 5.
- FIG. 8: is a graph showing a first example of the correlation between the ear cavity temperature and the auricle temperature at the time of an exercise load of 60 % in FIG. 5.
- FIG. 9: is a graph showing a second example of the correlation between the ear cavity temperature and the auricle temperature at the time of the exercise load of 60 % for a different biological body from that in FIG. 8.
- FIG. 10: is a graph showing a third example of the correlation between the ear cavity temperature and the auricle temperature at the time of the exercise load of 60 % for a different biological body from those in FIG. 8 and FIG. 9.
- FIG. 11: is a graph showing a fourth example of the correlation between the ear cavity temperature and the auricle temperature at the time of the exercise load of 60 % for a different biological body from those in FIG. 8 to FIG. 10.
- FIG. 12: is a graph showing temporal changes in temperatures of other sites of the biological body.
- FIG. 13: is a graph showing estimated behaviors of measurement results of sensors when the heatstroke prevention device according to Embodiment 1 is used with rise in environment temperature.
- FIG. 14: is a block diagram showing an example of the configuration of a heatstroke prevention device according to Embodiment 2.
- FIG. 15: is a functional block diagram showing an example of the configuration of a controller in FIG. 14.
- FIG. 16: is a graph for describing the relation between the auricle temperature and the ear cavity temperature when an auricle is cooled.
- FIG. 17: is a block diagram showing an example of the configuration of a heatstroke prevention system according to Embodiment 3.

### Description of Embodiments

### Embodiment 1

A heatstroke prevention device according to Embodiment 1 of the present invention will be described below. The heatstroke prevention device is worn by a biological body, and predicts the condition of the biological body on the basis of a detection result of a thermo-hygro sensor. In particular, in Embodiment 1, the heatstroke prevention device predicts the possibility of development of a heatstroke on the basis of the auricle temperature of the biological body.

### Configuration of Heatstroke Prevention Device 1

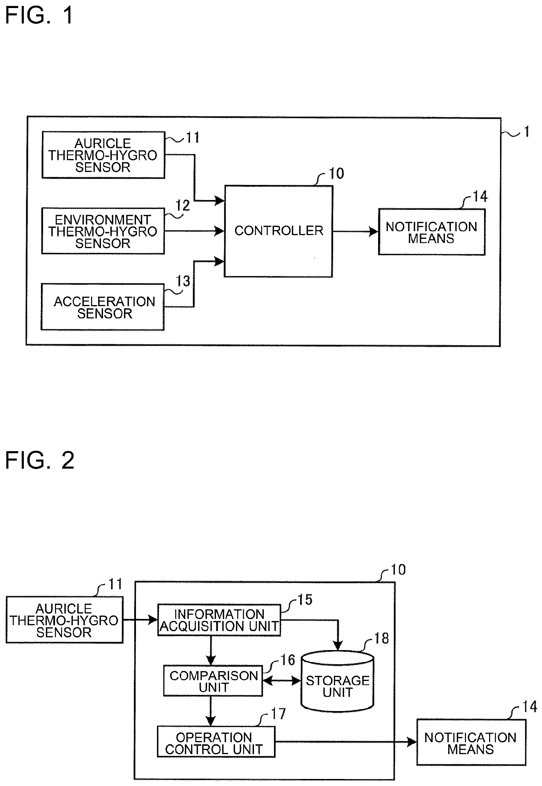

FIG. 1 is a block diagram showing an example of the configuration of a heatstroke prevention device 1 according to Embodiment 1. As shown in FIG. 1, the heatstroke prevention device 1 includes a controller 10 and an auricle thermo-hygro sensor 11. Further, the heatstroke prevention device 1 includes an environment thermo-hygro sensor 12, an acceleration sensor 13, and a notification means 14, as arbitrary components.

The auricle thermo-hygro sensor 11 measures the temperature and humidity of the auricle of the biological body. In Embodiment 1, the auricle thermo-hygro sensor 11 measures the temperature of the auricular lobule (earlobe) of the auricle, as the auricle temperature. The measurement spot is not limited to the auricular lobule, and may be any spot of the auricle. Preferably, the measurement spot may be any site that facilitates wearing of a sensor of a simple configuration such as a clip type and that does not have sweat glands, such as the ear conch and the auricular lobule.

A manner in which the auricle thermo-hygro sensor 11 is attached is not limited as long as the auricle thermo-hygro sensor 11 is attachable to the auricle. Examples of preferred shapes for attaching the auricle thermo-hygro sensor 11 include a clip shape pinching the auricular lobule, and a cover shape in which the whole of the auricle is covered along the outer edge of the ear conch. In this case, it is preferable that the clip and the cover be made from a lightweight material and have shapes insusceptible to the ambient temperature at the periphery of the auricle thermo-hygro sensor 11, because the improvement of the measurement accuracy is expected. Examples of the lightweight material insusceptible to the ambient temperature include foamed silicon. Although the heatstroke prevention device 1 may be attached to the biological body in any manner, the heatstroke prevention device 1 may be attached to a hat, a helmet, a headband, or eye glasses, for example.

The environment thermo-hygro sensor 12 measures the temperature and humidity of the ambient air. In Embodiment 1, in the case where the environment thermo-hygro sensor 12 is provided in the heatstroke prevention device 1, the environment thermo-hygro sensor 12 measures the temperature in the environment of a user, as an environment temperature. In this case, the heatstroke prevention device 1 may use the environment temperature measured by the environment thermo-hygro sensor 12 and the auricle temperature measured by the auricle thermo-hygro sensor 11, for the determination of the development of the heatstroke described later.

When the auricle temperature of the auricle thermo-hygro sensor 11 and the environment temperature of the environment thermo-hygro sensor 12 continue to have the same value, the heatstroke prevention device 1 is configured to estimate that the user does not wear the heatstroke prevention device 1. In this case, the heatstroke prevention device 1 is configured to prompt the user to wear the heatstroke prevention device 1, through a heatstroke prevention system described later.

The acceleration sensor 13 measures the acceleration of the biological body in each of three axial directions perpendicular to each other, for example, in each of an X direction, a Y direction, and a Z direction. In Embodiment 1, in the case where the acceleration sensor 13 is provided in the heatstroke prevention device 1, the acceleration sensor 13 is used for the estimation of the state of the user. Specifically, on the basis of the acceleration measured by the acceleration sensor 13, the heatstroke prevention device 1 is configured to estimate whether the user is moving, or whether the user has fallen down. The resultant force of three axial forces measured by the acceleration sensor 13 is an index allowing the presumption that the user is performing some sort of motion, and therefore, is defined as an active mass hereinafter.

When the acceleration measured by the acceleration sensor 13 is fully stable, the heatstroke prevention device 1 is configured to estimate that the user does not wear the heatstroke prevention device 1. In this case, the heatstroke prevention device 1 is configured to prompt the user to wear the heatstroke prevention device 1, through the heatstroke prevention system described later.

The controller 10 acquires a variety of sensor information measured by the auricle thermo-hygro sensor 11, the environment thermo-hygro sensor 12, and the acceleration sensor 13, at a preset timing, and determines the possibility of the development of the heatstroke on the basis of the acquired sensor information. Then, the controller 10 controls the notification means 14 on the basis of the determination result.

FIG. 2 is a functional block diagram showing an example of the configuration of the controller 10 in FIG. 1. As shown in FIG. 2, the controller 10 includes an information acquisition unit 15, a comparison unit 16, an operation control unit 17, and a storage unit 18. The controller 10 uses software on an arithmetic device such as a microcomputer, so that various functions are performed. Alternatively, the controller 10 is hardware such as circuit devices to perform various functions.

The information acquisition unit 15 acquires the information on the auricle temperature measured by the auricle thermo-hygro sensor 11, at a preset timing. The acquired information on the auricle temperature is transmitted to the comparison unit 16, and is transmitted to the storage unit 18 to be stored in the storage unit 18. The comparison unit 16 compares the auricle temperature acquired by the information acquisition unit 15 and a threshold stored in the storage unit 18, and determines the possibility of the development of the heatstroke. The threshold is a threshold for the determination of whether there is the possibility of the development of the heatstroke, and is previously decided by experiments or other methods.

The operation control unit 17 controls the operation of the notification means 14 on the basis of the determination result of the comparison unit 16. The storage unit 18 is, for example, a flash memory and stores a variety of information. The storage unit 18 stores the threshold to be used by the comparison unit 16. Further, the storage unit 18 stores the auricle temperature acquired by the information acquisition unit 15, and stores information providing the determination result of the comparison unit 16.

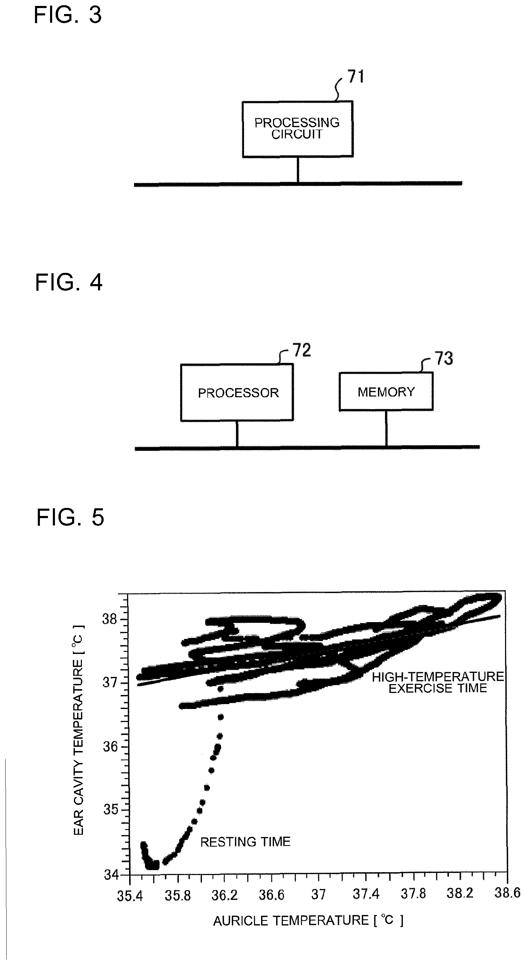

FIG. 3 is a hardware configuration diagram showing an example of the configuration of the controller 10 in FIG. 2. In the case where the various functions of the controller 10 are performed by hardware, the controller 10 in FIG. 2 is composed of a processing circuit 71, as shown in FIG. 3. In the controller 10 in FIG. 2, the functions of the information acquisition unit 15, the comparison unit 16, the operation control unit 17, and the storage unit 18 are performed by the processing circuit 71.

In the case where the functions are performed by hardware, examples of the processing circuit 71 include a single circuit, a composite circuit, a programed processor, a parallel programed processor, an application specific integrated circuit (ASIC), a field-programmable gate array (FPGA), and a combination of them. The controller 10 may perform the functions of the information acquisition unit 15, the comparison unit 16, the operation control unit 17, and the storage unit 18, with respective processing circuits 71, or may perform the functions of the units with one processing circuit 71.

FIG. 4 is a hardware configuration diagram showing another example of the configuration of the controller 10 in FIG. 2. In the case where the various functions of the controller 10 are performed by software, the controller 10 in FIG. 2 includes a processor 72 and a memory 73 as shown in FIG. 4. In the controller 10, the functions of the information acquisition unit 15, the comparison unit 16, the operation control unit 17, and the storage unit 18 are performed by the processor 72 and the memory 73.

In the case where the functions are performed by software, the functions of the information acquisition unit 15, the comparison unit 16, the operation control unit 17, and the storage unit 18 are performed in the controller 10, by software, firmware, or a combination of the software and the firmware. The software and the firmware are described as programs, and are stored in the memory 73. The processor 72 reads and executes the programs stored in the memory 73, and thereby performs the functions of the units.

As the memory 73, for example, a volatile or non-volatile semiconductor memory such as a random access memory (RAM), a read only memory (ROM), a flash memory, an erasable and programmable ROM (EPROM), and an electrically erasable and programmable ROM (EEPROM) are used. Further, as the memory 73, for example, a detachable recording medium such as a magnetic disc, a flexible disc, an optical disc, a compact disc (CD), a mini disc (MD), and a digital versatile disc (DVD) may be used.

The notification means 14 in FIG. 1 gives a notice that there is the possibility of the development of the heatstroke on the basis of the determination result of the controller 10. As the notification means 14, for example, an LED, a speaker, or other devices are used. In the case where the notification means 14 is the LED, the determination result is shown by the LED, for example, turning on, blinking, and turning off. In the case where the notification means 14 is the speaker, the determination result is given by voice and sound.

### Relation between Development of Heatstroke and Auricle Temperature

First, the relation between the development of the heatstroke and the auricle temperature will be described. It is widely known that the heatstroke is developed by the rise in the brain temperature that is the deep body temperature of the biological body. Further, it is known that the brain temperature has a high correlation to the ear cavity temperature, and it is possible to obtain a temperature close to the brain temperature by measuring the ear cavity temperature.

When the ear cavity temperature is measured, it is necessary to measure the ear cavity temperature, for example, by inserting a thermo-hygro sensor such as an eardrum thermometer into the ear cavity. Exercise or working in the state where such a thermo-hygro sensor is worn is very bothersome to the user. Further, the insertion of the thermo-hygro sensor into the ear cavity closes the ear cavity, and therefore there also arises a problem of difficulty in hearing external sound unless the thermo-hygro sensor has a special configuration. Therefore, it is difficult to determine the possibility of the development of the heatstroke while measuring the ear cavity temperature during exercise or working.

Hence, the inventors have studied a site that has a high correlation to the brain temperature instead of the ear cavity temperature and to which the thermo-hygro sensor can be simply attached. Then, as a result of the study, the inventors have found that the auricle temperature has a high correlation to the brain temperature.

FIG. 5 is a graph showing an example of the correlation between the ear cavity temperature and the auricle temperature from a resting time to an exercise ending time in a room in which a hot environment (wet-bulb globe temperature (WBGT) 31 °C) is provided. In the example shown in FIG. 5, the ordinate axis represents the ear cavity temperature, and the abscissa axis represents the auricle temperature. In this example, the ear cavity temperature is measured at the left ear of the biological body, and the auricle temperature is measured at a front portion of the auricular lobule of the left ear.

The example shown in FIG. 5 shows the correlation between the ear cavity temperature and the auricle temperature in a succession of states including the start of exercise, the rise in exercise load, and the end of the exercise. As shown in FIG. 5, in the whole from the resting time to the exercise ending time, the correlation between the auricle temperature and the ear cavity temperature close to the brain temperature is recognized at some portions, and is not recognized at some portions. Therefore, it is difficult to determine whether there is a high correlation between the ear cavity temperature and the auricle temperature.

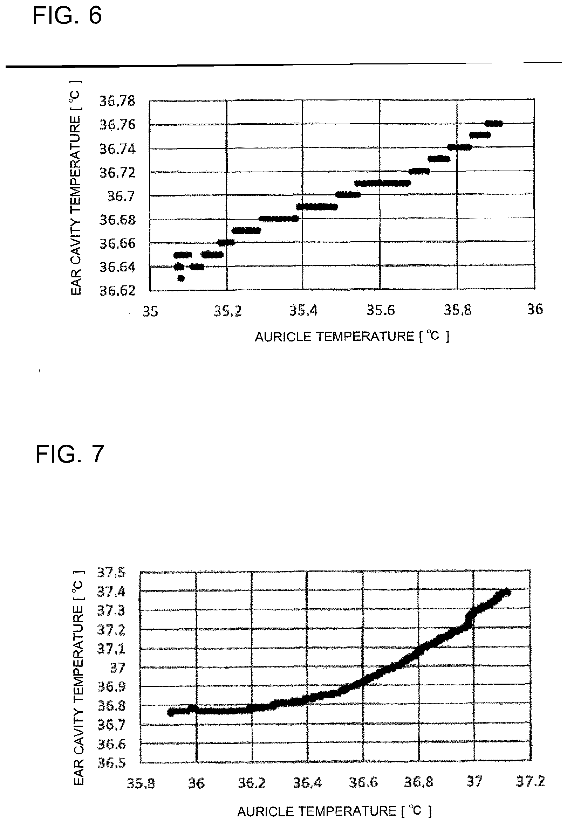

FIG. 6 is a graph showing an example of the correlation between the ear cavity temperature and the auricle temperature during the resting time in FIG. 5. The example in FIG. 6 shows a case where the biological body stays in a resting state for 5 minutes in the room in which the hot environment is provided. FIG. 7 is a graph showing an example of the correlation between the ear cavity temperature and the auricle temperature at the time of the warm-up in FIG. 5.

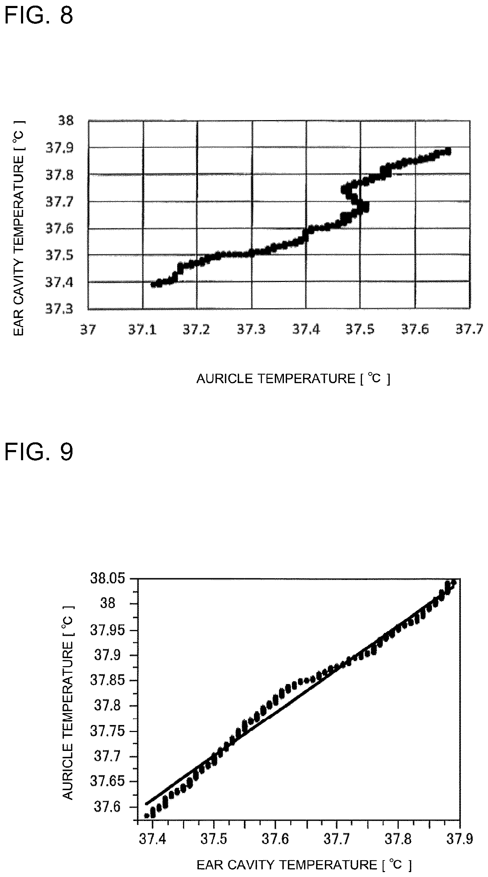

The example in FIG. 7 shows a case where the biological body starts the exercise in the room and is in a light exercise load state. FIG. 8 is a graph showing a first example of the correlation between the ear cavity temperature and the auricle temperature at the time of an exercise load of 60 % in FIG. 5. The example of FIG. 8 shows a case where the biological body is performing the exercise at the exercise load of 60 %. In each of the examples shown in FIG. 6 to FIG. 8, the ordinate axis represents the ear cavity temperature, and the abscissa axis represents the auricle temperature.

Unlike FIG. 5, in the examples shown in FIG. 6 to FIG. 8, in a period from the time of the resting state of the biological body in the room in which the hot environment is provided to the time of the state of the exercise load of the 60 %, the auricle temperature tends to rise as the ear cavity temperature rises. Further, as shown in FIG. 8, it is found that there is a high correlation of 1:1 between the rise rate of the ear cavity temperature and the rise rate of the auricle temperature at the time of the exercise load of 60 %.

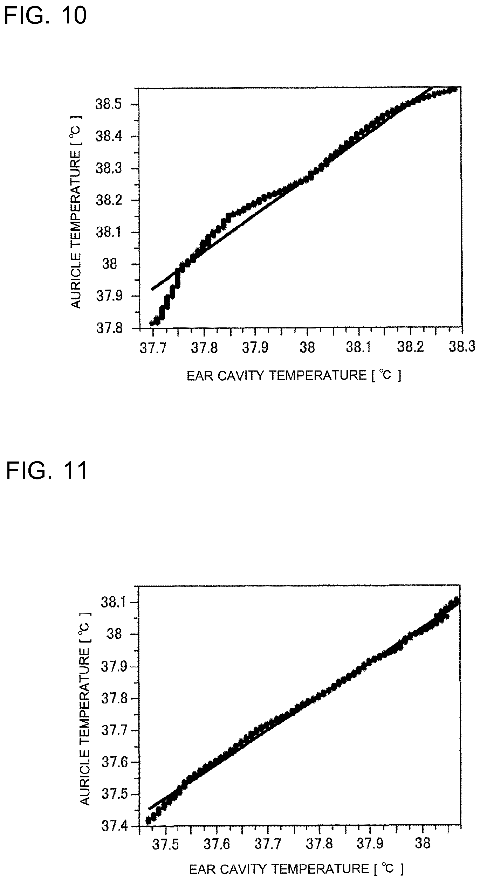

FIG. 9 is a graph showing a second example of the correlation between the ear cavity temperature and the auricle temperature at the time of the exercise load of 60 %, which is the same as that in FIG. 8, for a different biological body from that in FIG. 8. FIG. 10 is a graph showing a third example of the correlation between the ear cavity temperature and the auricle temperature at the time of the exercise load of 60 %, for a different biological body from those in FIG. 8 and FIG. 9.

FIG. 11 is a graph showing a fourth example of the correlation between the ear cavity temperature and the auricle temperature at the time of the exercise load of 60 % for a different biological body from those in FIG. 8 to FIG. 10. FIG. 8 to FIG. 11 each shows the correlation between the ear cavity temperature and the auricle temperature in the case where the exercise load is 60 %, for the corresponding one of biological bodies different from each other. In each of the examples shown in FIG. 9 to FIG. 11, the ordinate axis represents the auricle temperature, and the abscissa axis represents the ear cavity temperature. Further, in FIG. 9 to FIG. 11, an approximate straight line having a slope of about 1 is put as a reference.

As shown in FIG. 8 to FIG. 11, at the time of the exercise load of 60 %, the auricle temperature rises at a rate of 1:1 as the ear cavity temperature rises. It is found that there is a high correlation between the ear cavity temperature and the auricle temperature with no individual difference.

Thus, from results shown in FIG. 5 to FIG. 11, it is found that the auricle temperature and the ear cavity temperature have no correlation during the resting time and have a correlation during the exercise. It is found that the auricle temperature and the ear cavity temperature have a high correlation, in particular, at the time of the exercise load of 60 %.

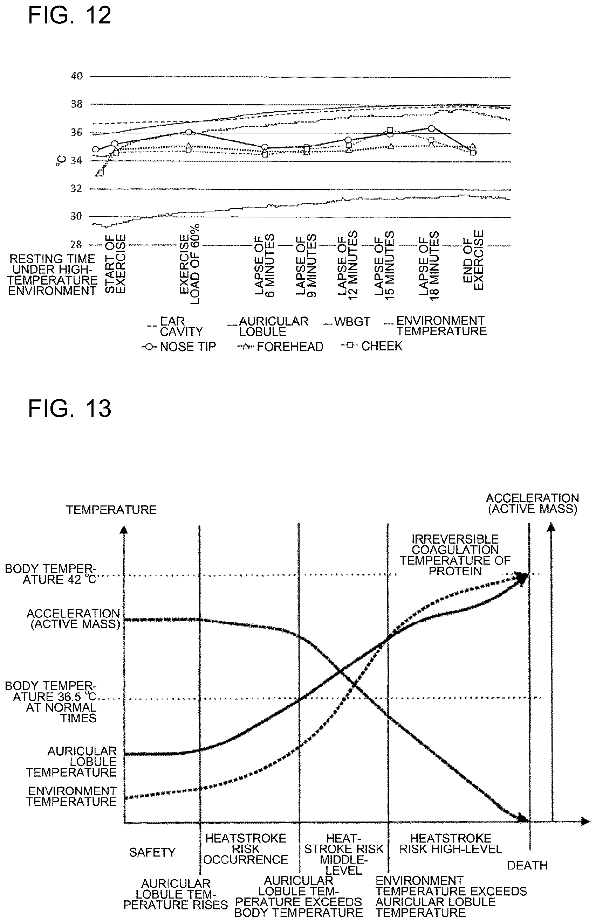

Next, relations between temperatures of other sites of the biological body and the ear cavity temperature will be described as a comparative example against the auricle temperature. FIG. 12 is a graph showing an example of temporal changes in temperatures of other sites of an identical biological body under a high-temperature environment in states including the start of the exercise at the resting time, the continuation of the exercise load of 60 %, and the end of the exercise.

This example shows comparisons of the auricle temperature and the ear cavity temperature with the temperature of the nose tip, the temperature of the cheek, and the temperature of the forehead, which are said to be relatively close to the brain temperature, as sites other than the auricle and the ear cavity. In FIG. 12, the ordinate axis represents the temperatures of the sites, and the abscissa axis represents the time from the resting time to the exercise ending time.

As shown in FIG. 12, the temperatures of the nose tip, the cheek, and the forehead hardly change from the start of the exercise even in the state of the continuation of the exercise load of 60 %. On the other hand, the auricle temperature rises in proportion to the ear cavity temperature. Thus, it is understood that when the ear cavity temperature having a high correlation to the brain temperature rises at the time of the exercise load, the auricle temperature rises such that the auricle temperature properly corresponds to the ear cavity temperature, but the temperatures of the nose tip, the cheek, and the forehead, which are said to be relatively close to the brain temperature in addition to the auricle temperature, do not exactly correspond to the ear cavity temperature.

This reveals that the auricle temperature has the highest correlation to the ear cavity temperature at the time of the exercise load. The inventors estimate that the reason why the auricle temperature exactly corresponds to the ear cavity temperature is that the auricle has fewer sweat glands than do other sites of the biological body and is less affected by the temperature regulation with sweat.

Thus, from the knowledge newly discovered by the inventors, during the exercise, the auricle temperature has a high correlation to the ear cavity temperature, and therefore it is possible to almost exactly determine the condition of the brain temperature by measuring the auricle temperature. Further, the auricle allows the biological body to easily wear the sensor, unlike the ear cavity. Hence, the heatstroke prevention device 1 according to Embodiment 1 determines the possibility of the development of the heatstroke on the basis of the measurement result of the auricle temperature, and performs a heatstroke prevention process for preventing the heatstroke.

### Heatstroke Prevention Process

The heatstroke prevention process by the heatstroke prevention device 1 will be described with reference to FIG. 2. First, the information acquisition unit 15 of the controller 10 acquires the information on the auricle temperature measured by the auricle thermo-hygro sensor 11, at a preset timing. The information acquisition unit 15 transmits the acquired information on the auricle temperature to the comparison unit 16, and causes the storage unit 18 to store the information on the auricle temperature.

The comparison unit 16 reads the threshold set for the auricle temperature, from the storage unit 18, compares the information on the auricle temperature transmitted from the information acquisition unit 15 and the threshold read from the storage unit 18, and determines whether the auricle temperature is higher than the threshold.

In the case where the auricle temperature is higher than the threshold, the comparison unit 16 determines that there is the possibility that the biological body develops the heatstroke. On the other hand, in the case where the auricle temperature is lower than or equal to the threshold, the comparison unit 16 determines that there is a low possibility that the biological body develops the heatstroke. Then, the comparison unit 16 transmits the determination result to the operation control unit 17, and causes the storage unit 18 to store the determination result.

In the case where the determination result of the comparison unit 16 indicates that there is a high possibility of the development of the heatstroke, the operation control unit 17 controls the notification means 14, and causes the notification means 14 to give a notice that there is the possibility of the development of the heatstroke. Thereby, the notice of the possibility of the development of the heatstroke is given to the user.

Here, a setting method for the threshold will be described. A problem caused by the heatstroke is a damage to various organs due to the rise in the brain temperature. The heat is usually dissipated by sweating and other bodily functions, and thereby the brain temperature is maintained. However, it is said that the brain temperature rises when the heat is not appropriately dissipated by various causes such as an excessively high environment temperature. Further, it is known that the irreversible coagulation temperature of the protein in the human body is around 42 °C.

Furthermore, the heatstroke causes decrease in active mass, and when symptoms worsen and consciousness is lost, it is not possible to move. From such well-known phenomena and other bases, the inventors estimate that the measurement results of the auricle thermo-hygro sensor 11, the environment thermo-hygro sensor 12, and the acceleration sensor 13 behave as shown in FIG. 13 when the user of the heatstroke prevention device 1 acts under a hot environment.

FIG. 13 is a graph showing estimated behaviors of the measurement results obtained by the sensors when the heatstroke prevention device 1 according to Embodiment 1 is used with rise in the environment temperature. In the example shown in FIG. 13, the temperature of the auricular lobule is measured as the auricle temperature.

In a safety region shown in FIG. 13, the environment temperature is lower than the auricular lobule temperature. When there is no body abnormality such as common cold, the auricular lobule temperature is lower than the body temperature (36.5 °C) at normal times. The user can act as usual, and therefore the active mass that is the resultant force of the three axial forces of the acceleration sensor 13 is high. In this state, the heat produced by the user is also allowed to be normally dissipated, and the auricular lobule temperature is maintained.

In a heatstroke risk occurrence region shown in FIG. 13, the environment temperature rises, and the auricular lobule temperature in the stable state also rises. The auricular lobule temperature rises to the same level as the body temperature (36.5 °C) at normal times. In experiments by the inventors, in this region, the circulatory blood volume to the auricular lobule increased, possibly because the dissipation of the heat of the brain was necessary. Further, in this region, the user starts to feel tired because of the heat, and the active mass decreases.

In a heatstroke risk middle-level region and a subsequent region shown in FIG. 13, the heatstroke is estimated from well-known phenomena and other bases. In the heatstroke risk middle-level region, the environment temperature further rises. At the start of the heatstroke risk middle-level region, the auricular lobule temperature exceeds the body temperature (36.5 °C) at normal times, and at the end of the heatstroke risk middle-level region, the environment temperature exceeds the auricular lobule temperature.

In this region, as the environment temperature gets close to the auricular lobule temperature, the heat dissipation is difficult, and for example, when the user is acting, the heat dissipation by sweating and other bodily functions reaches the limit. Thereby, it is estimated that the user further feels tired because of the heat and the active mass significantly decreases.

It is estimated that a heatstroke risk high-level region appears after the heatstroke risk middle-level region. At the start of the heatstroke risk high-level region, the environment temperature exceeds the auricular lobule temperature, and at the end of the heatstroke risk high-level region, the auricular lobule temperature reaches 42 °C, which is the irreversible coagulation temperature of the protein. In this region, it is impossible to perform the heat dissipation from the auricular lobule, and it is difficult to decrease the brain temperature by cooling the brain through sweating and other bodily functions.

In the heatstroke risk high-level region, the user can hardly act, and in some cases, the user falls down, so that the user cannot move. When the transport to a place where the environment temperature is low and the brain cooling from the exterior are not actively performed, user's life is in danger. As the denaturation temperature of the protein is 42 °C, when the body temperature exceeds 42 °C, there is a high possibility that user's life is in danger or the user has a serious aftereffect.

From the above description, in Embodiment 1, it is preferable that the threshold of the auricular lobule temperature (auricle temperature) be within the heatstroke risk occurrence region. Therefore, for example, the threshold may be set to such a temperature that the auricular lobule temperature rises to a temperature close to the body temperature (36.5 °C) at normal times.

Alternatively, the threshold may be set on the basis of the rise rate of the auricular lobule temperature, or may be set on the basis of the combination of the active mass and the auricular lobule temperature. The setting of the threshold is not limited to the above examples, and may be made by analyzing the data accumulated about the heatstroke using an artificial intelligence (AI) and selecting the most effective value from the analysis result.

Furthermore, the heatstroke is developed by a mix of many causes. Hence, the threshold for the determination of the possibility of the development of the heatstroke may be set for numerical values obtained using various parameters that are estimated to be associated with the heatstroke. Specifically, for example, the surrounding environment temperature, auricular lobule temperature, humidity, atmospheric pressure, labor time (or exercise time or operation time), working time, active mass, sex, age, and individual difference of a person who is actually working are set as the parameters associated with the heatstroke.

After the parameters associated with the heatstroke are set, heatstroke indexes representing magnitudes of the association with the heatstroke are set for the values of the parameters. For example, the heatstroke index is set such that the numerical value differs for each section when the value of the parameter is divided into a plurality of sections. When the parameters are quantified in this manner, the heatstroke indexes of the parameters are summed. Then, the threshold is set for the sum of the heatstroke indexes.

The plurality of set parameters is different from each other in the closeness of the association with the heatstroke. Accordingly, when heatstroke indexes are set for a plurality of various parameters, weighting is performed for each of the parameters, depending on the closeness of the association with the heatstroke.

For example, the auricular lobule temperature as a parameter is used when the brain temperature is evaluated. It is said that the brain temperature has the closest association with the heatstroke. Accordingly, the heaviest weight is given to the auricular lobule temperature. Further, the possibility of the development of the heatstroke increases as the environment temperature increases, and therefore a certain degree of weight is given to the environment temperature. Similarly, the possibility of the development of the heatstroke increases as the humidity increases, and therefore a certain degree of weight is given to the humidity as well. Furthermore, the resistance against the heatstroke differs depending on sex difference such as difference between male and female, and therefore a different heatstroke index is given to each sex.

In the case where the environment temperature, the auricular lobule temperature, the humidity, and the sex are set as parameters, the relation between the parameters and the heatstroke indexes is shown in Table 1. In this case, each heatstroke index is an example of a value estimated to be appropriate by the inventors, from experiment results from the resting time to the exercise ending time in the room in which the hot environment is provided, in consideration of weighting. The parameter kinds and heatstroke indexes shown in Table 1 are examples, and the parameter kind and the heatstroke index are not limited to Table 1.

**Table 1**

| Environment Temperature | Value | Auricular Lobule Temperature | Value | Humidity | Value | Sex | Value |
|---|---|---|---|---|---|---|---|
| 38 °C or higher | 15 | 41 °C or higher | 30 | 90 % or higher | 8 | Male | 0 |
| 37 °C or higher and lower than 38 °C | 13 | 40 °C or higher and lower than 41 °C | 20 | 80 % or higher and lower than 90% | 7 | Female | 2 |
| 35 °C or higher and lower than 37 °C | 12 | 39 °C or higher and lower than 40 °C | 14 | 75 % or higher and lower than 80% | 5 | — | |
| 33 °C or higher and lower than 35 °C | 8 | 38 °C or higher and lower than 39 °C | 12 | 70 % or higher and lower than 75 % | 4 | | |
| 31 °C or higher and lower than 33 °C | 5 | 37 °C or higher and lower than 38 °C | 6 | 60 % or higher and lower than 70% | 3 | | |
| 29 °C or higher and lower than 31 °C | 3 | 36 °C or higher and lower than 37 °C | 2 | 50 % or higher and lower than 60% | 2 | | |
| 26 °C or higher and lower than 29 °C | 2 | 35 °C or higher and lower than 36 °C | 0 | 30 % or higher and lower than 50% | 0 | | |
| Lower than 26 °C | 0 | Lower than 35 °C | 0 | Lower than 30% | 0 | | |

The parameters that are estimated to be associated with the heatstroke are not limited to the above examples, and the number of the parameters may be increased or may be decreased. The heatstroke index for each parameter may be modified to an optimal value to the extent possible on the basis of much data to be accumulated, as necessary.

In Embodiment 1, the controller 10 of the heatstroke prevention device 1 derives the heatstroke index for each parameter on the basis of sensor information measured by various sensors or other devices. In the derivation of the heatstroke index, it is allowable to store, for example, a table providing the correspondence relation between the values of the parameters shown in Table 1 and the heatstroke indexes, in the storage unit 18, and to refer to the table and the sensor information, or it is allowable to use a preset arithmetic expression or other settings.

The controller 10 sums the heatstroke indexes for the parameters, and determines the possibility of the development of the heatstroke on the basis of the sum of the heatstroke indexes. For example, the controller 10 makes a determination of "danger" when the sum of the heatstroke indexes is 36 or more, and makes a determination of "suggestion of stop of work (exercise or motion)" when the sum of the heatstroke indexes is 30 or more and 35 or less. Further, the controller 10 makes a determination of "close watch (alert)" when the sum of the heatstroke indexes is 25 or more and 29 or less, and makes a determination of "watch is necessary" when the sum of the heatstroke indexes is 21 or more and 24 or less.

The controller 10 makes a determination of "attention is necessary" when the sum of the heatstroke indexes is 15 or more and 20 or less, and makes a determination of "measure preparation is necessary" when the sum of the heatstroke indexes is 12 or more and 14 or less. The controller 10 makes a determination of "safety" when the sum of the heatstroke indexes is 8 or more and 11 or less, and makes a determination of "no problem" when the sum of the heatstroke indexes is 7 or less. In this manner, the controller 10 determines the possibility of the development of the heatstroke depending on the heatstroke indexes, and performs operation depending on the possibility.

Environment data such as the temperature, humidity, and atmospheric pressure for the last several days, environment statistic data about the measurement place for several years, and accumulative heat stress data for which the labor time (or exercise time or operation time), the working time, the active mass, and other factors are considered may be used as data for the prediction of the heatstroke, in addition to the data about the current state.

By using a variety of data estimated to be associated with the heatstroke in this manner, it is possible to more exactly determine the possibility of the development of the heatstroke.

As described above, in the heatstroke prevention device 1 according to Embodiment 1, it is determined whether there is the possibility of the development of the heatstroke on the basis of the auricle temperature of the biological body measured by the auricle thermo-hygro sensor 11 or on the basis of a complicated factor including the auricle temperature, the environment temperature, and other factors. In the case where it is determined that there is the possibility of the development of the heatstroke, the notice of the determination result is given to the biological body.

In Embodiment 1, as the auricle temperature having a correlation to the brain temperature is measured, it is possible to determine the possibility of the development of the heatstroke easily and surely, and to prevent the heatstroke. Further, in Embodiment 1, the temperature of the auricle is measured, unlike the case where the temperature of the ear cavity is measured, and therefore it is possible to measure the temperature during exercise or working while the biological body is not conscious of the measurement.

In Embodiment 1, the auricle thermo-hygro sensor 11 measures the temperature of the auricular lobule, which is a part of the auricle of the biological body. The temperature of the auricular lobule has a high correlation to the brain temperature, and therefore, by measuring the temperature of the auricular lobule, it is possible to more surely determine the possibility of the development of the heatstroke.

### Embodiment 2

Embodiment 2 of the present invention will be described. Embodiment 2 is different from Embodiment 1 in that the biological body is cooled in the case where there is the possibility of the development of the heatstroke. In the following description, parts in common to Embodiment 1 are denoted by the same reference signs, and detailed descriptions are omitted.

### Configuration of Heatstroke Prevention Device 1

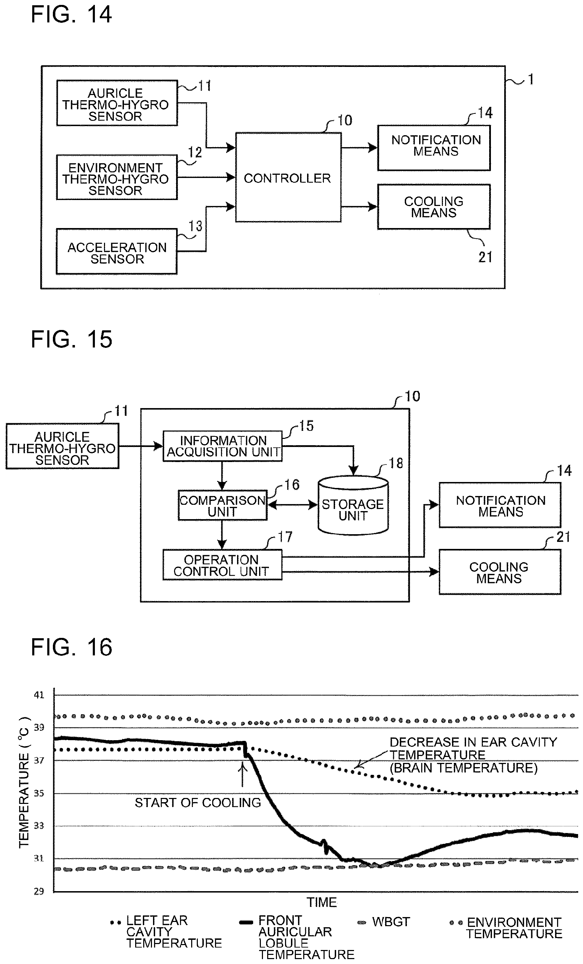

FIG. 14 is a block diagram showing an example of the configuration of a heatstroke prevention device 1 according to Embodiment 2. As shown in FIG. 14, the heatstroke prevention device 1 includes the controller 10, the auricle thermo-hygro sensor 11, the environment thermo-hygro sensor 12, the acceleration sensor 13, the notification means 14 and a cooling means 21.

The cooling means 21 is provided at the periphery of the auricle of the biological body, and cools the auricle. As the cooling means 21, a fan, a Peltier element, or other components that electrically operate and cool the auricle may be used, or a cooling gel or other components physically cool the periphery may be used.

Similarly to Embodiment 1, the controller 10 determines the possibility of the development of the heatstroke on the basis of a variety of sensor information. Then, the controller 10 controls the notification means 14 and the cooling means 21 on the basis of the determination result.

FIG. 15 is a functional block diagram showing an example of the configuration of the controller 10 in FIG. 14. As shown in FIG. 15, the controller 10 includes the information acquisition unit 15, the comparison unit 16, the operation control unit 17, and the storage unit 18, similarly to Embodiment 1. In Embodiment 2, in the case where the comparison unit 16 determines that there is the possibility of the development of the heatstroke, the operation control unit 17 controls and operates the notification means 14 and the cooling means 21.

### Cooling of Auricle

The cooling of the auricle will be described. As described above, the heatstroke is developed because of the rise in the brain temperature. Therefore, in the case where it is determined that there is the possibility of the development of the heatstroke as a result of the measurement of the auricle temperature, it is necessary to decrease the brain temperature for suppressing the heatstroke.

Here, the high correlation between the auricle temperature and the brain temperature means that it is possible to determine the condition of the brain temperature by measuring the auricle temperature, and that the auricle temperature changes with the change in the brain temperature. That is, in the case where the brain temperature rises because of the development of the heatstroke, it is possible to decrease the brain temperature by cooling the auricle and decreasing the auricle temperature.

FIG. 16 is a graph for describing the relation between the auricle temperature and the ear cavity temperature when the auricle is cooled. As shown in FIG. 16, when the cooling of the auricle is started, the auricle temperature decreases, and the ear cavity temperature gradually decreases, accordingly. As the ear cavity temperature has a correlation to the brain temperature as described above, the decrease in the ear cavity temperature is reasonably regarded as the decrease in the brain temperature. Accordingly, by cooling the auricle and decreasing the auricle temperature, it is possible to decrease the brain temperature, and to reduce the possibility of the development of the heatstroke.

Hence, in the case where it is determined that there is the possibility of the development of the heatstroke, the heatstroke prevention device 1 according to Embodiment 2 cools the auricle to decrease the brain temperature.

### Heatstroke Prevention Process

The heatstroke prevention process by the heatstroke prevention device 1 will be described with reference to FIG. 15. First, the information acquisition unit 15 of the controller 10 acquires the information on the auricle temperature measured by the auricle thermo-hygro sensor 11, at a preset timing. The information acquisition unit 15 transmits the acquired information on the auricle temperature to the comparison unit 16, and causes the storage unit 18 to store the information on the auricle temperature.

The comparison unit 16 reads the threshold set for the auricle temperature, from the storage unit 18, compares the information on the auricle temperature transmitted from the information acquisition unit 15 and the threshold read from the storage unit 18, and determines whether the auricle temperature is higher than the threshold.

In the case where the auricle temperature is higher than the threshold, the comparison unit 16 determines that there is the possibility that the biological body develops the heatstroke. On the other hand, in the case where the auricle temperature is lower than or equal to the threshold, the comparison unit 16 determines that there is a low possibility that the biological body develops the heatstroke. Then, the comparison unit 16 transmits the determination result to the operation control unit 17, and causes the storage unit 18 to store the determination result.

In the case where the determination result of the comparison unit 16 indicates that there is a high possibility of the development of the heatstroke, the operation control unit 17 controls the notification means 14, and causes the notification means 14 to give the notice that there is the possibility of the development of the heatstroke. Thereby, the notice of the possibility of the development of the heatstroke is given to the user.

Further, in the case where the determination result of the comparison unit 16 indicates that there is a high possibility of the development of the heatstroke, the operation control unit 17 controls the cooling means 21 such that the cooling means 21 cools the auricle. Thereby, it is possible to decrease the brain temperature of the user for which it is determined that there is the possibility of the development of the heatstroke, and to decrease the possibility of the development of the heatstroke.

As described above, in the heatstroke prevention device 1 according to Embodiment 2, in the case where it is determined that there is the possibility of the development of the heatstroke, the periphery of the auricle is cooled by the cooling means 21. Thereby, the brain temperature decreases, and therefore it is possible to inhibit the development of the heatstroke in the biological body.

In the heatstroke prevention process, the measure in the case where it is determined that there is the possibility that the biological body develops the heatstroke is not limited to the cooling of the auricle. For example, it is allowable to give a notice to prompt the user to drink water, or it is allowable to give a notice to prompt the user to suspend user's work, move to a cool place, get some air, or cool each part of the body. When the user takes such a measure in accordance with the notice, the brain temperature decreases, and therefore it is also possible to inhibit the development of the heatstroke in the biological body.

### Embodiment 3

Embodiment 3 of the present invention will be described. Embodiment 3 is different from Embodiment 1 and Embodiment 2 in that the heatstroke prevention device 1 is configured as a system. In the following description, parts in common to Embodiment 1 and Embodiment 2 are denoted by the same reference signs, and detailed descriptions are omitted.

### Configuration of Heatstroke Prevention System 100

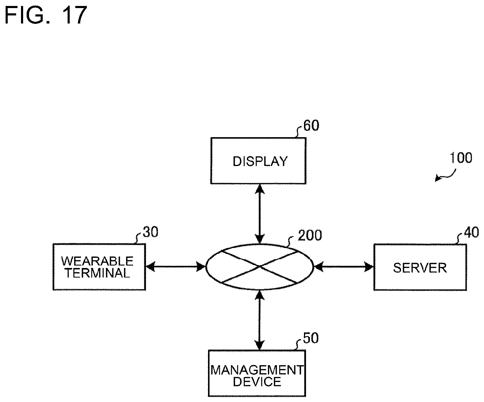

FIG. 17 is a block diagram showing an example of the configuration of a heatstroke prevention system 100 according to Embodiment 3. As shown in FIG. 17, the heatstroke prevention system 100 includes a wearable terminal 30, a server 40, a management device 50, and a display 60, and the wearable terminal 30, the server 40, the management device 50, and the display 60 are connected to a network 200 such as the internet. The wearable terminal 30 is connected to the network 200 by wireless. The server 40, the management device 50, and the display 60 are connected to the network 200 by wire or by wireless.

### Wearable Terminal 30

The wearable terminal 30 in FIG. 17 has the same configuration as the heatstroke prevention device 1 described in FIG. 14, except that the controller 10 includes a communication circuitry to perform communication with the network 200. In Embodiment 3, the controller 10 and the notification means 14 of the wearable terminal 30 may be configured as a compact device to be attached to a helmet of a worker at a construction site, for example.

In this case, the compact device on the helmet that includes the controller 10 and the notification means 14 are connected with the auricle thermo-hygro sensor 11 that is formed in a clip shape or other shapes and that pinches the auricular lobule of the worker at the construction site, by wire or by wireless. Functions other than the function of the auricle thermo-hygro sensor 11 such as the functions of the controller 10 and the notification means 14 of the wearable terminal 30 may be performed by programs implemented in a portable device carried by the worker at the construction site, as exemplified by a smartphone or a wearable terminal.

The communication circuitry performs communication with the server 40 and the management device 50 connected through the network 200, in accordance with a previously decided protocol. The communication circuitry transfers sensor information transmitted from various sensors to the server 40 and the management device 50. Further, the communication circuitry receives the determination result about the heatstroke transmitted from the network 200, and transmits the received determination result to the controller 10.

The controller 10 acquires a variety of sensor information measured by the auricle thermo-hygro sensor 11, the environment thermo-hygro sensor 12, and the acceleration sensor 13, at a preset timing, and transmits the acquired sensor information to the communication circuitry. Further, the controller 10 controls the notification means 14 and the cooling means 21 on the basis of the determination result about the heatstroke received from the server 40 through the communication circuitry.

The information acquisition unit 15 of the controller 10 acquires the information on the auricle temperature measured by the auricle thermo-hygro sensor 11, at a preset timing. The acquired information on the auricle temperature is transmitted to the communication circuitry, and is transmitted to the storage unit 18 to be stored in the storage unit 18. Further, the information acquisition unit 15 receives the determination result about the heatstroke from the server 40 through the communication circuitry, and transmits the determination result to the operation control unit 17.

The operation control unit 17 of the controller 10 controls and operates the notification means 14 and the cooling means 21 on the basis of the determination result received from the information acquisition unit 15.

### Server 40

The server 40 in FIG. 17 determines whether there is the possibility of the development of the heatstroke on the basis of the evaluated condition of the biological body based on measurement, and includes a communication circuitry, an arithmetic device, and a storage unit.

The communication circuitry of the server 40 performs communication with the wearable terminal 30, the management device 50, and the display 60 connected through the network 200, in accordance with a previously decided protocol. The communication circuitry of the server 40 receives the sensor information sent from the wearable terminal 30 through the network 200, and transmits the received sensor information to the arithmetic device of the server 40. Further, the communication circuitry of the server 40 transfers the determination result about the heatstroke determined by the arithmetic device of the server 40, to the wearable terminal 30, the management device 50, and the display 60.

The arithmetic device of the server 40 compares the auricle temperature included in the sensor information received from the wearable terminal 30 through the communication circuitry of the server 40 and the threshold of the auricle temperature stored in the storage unit of the server 40, and determines the possibility of the development of the heatstroke. The arithmetic device of the server 40 transmits information providing the determination result, to the communication circuitry of the server 40, and stores the information in the storage unit of the server 40.

The storage unit of the server 40 is, for example, a flash memory and stores a variety of information. The storage unit of the server 40 stores the threshold to be used by the arithmetic device of the server 40. Further, the storage unit of the server 40 stores the sensor information including the auricle temperature and received by the communication circuitry of the server 40, and stores the information providing the determination result of the arithmetic device of the server 40.

### Management Device 50

The management device 50 in FIG. 17 manages the condition of the biological body, and includes a communication circuitry, a controller, a display means, an operation unit, and a storage unit. For example, the management device 50 is a device to be installed in a management center at the construction site, and is used by a site supervisor to manage the prevention of the heatstroke of the worker.

The communication circuitry of the management device 50 performs communication with the wearable terminal 30 and the server 40 connected through the network 200, in accordance with a previously decided protocol. The communication circuitry of the management device 50 receives the determination result about the heatstroke determined by the arithmetic device of the server 40 through the network 200, and transmits the received determination result to the controller of the management device 50. Further, the communication circuitry of the management device 50 receives the sensor information sent from the wearable terminal 30 through the network 200, and transmits the received sensor information to the controller of the management device 50. Furthermore, the communication circuitry of the management device 50 receives a command from the controller of the management device 50, and sends the command to the wearable terminal 30 through the network 200.

The controller of the management device 50 stores the sensor information received from the wearable terminal 30 through the communication circuitry of the management device 50 and the determination result about the heatstroke received from the server 40, to the storage unit of the management device 50. Further, the controller of the management device 50 causes the display means of the management device 50 to display the received sensor information and determination result. Furthermore, the controller of the management device 50 issues a command for the wearable terminal 30 on the basis of an operation signal input when a manager operates the operation unit of the management device 50. The issued command is sent to the wearable terminal 30 through the communication circuitry of the management device 50.

The display means of the management device 50 is a well-known display or other devices, and displays the determination result about the heatstroke, or other information.

The operation unit of the management device 50 is provided with various well-known keys and other components to be used for operating the management device 50, and outputs operation signals corresponding to the operation of the keys and other components.

The storage unit of the management device 50 stores the sensor information including the auricle temperature and received by the communication circuitry of the management device 50, and stores the information providing the determination result about the heatstroke and received from the server 40.

### Display 60

The display 60 in FIG. 17 is a well-known display, and displays the determination result about the possibility of the development of the heatstroke after the processing by the server 40. For example, the display 60 may be a portable device or other devices carried by the worker at the construction site, as exemplified by a smartphone or a wearable terminal.

### Heatstroke Prevention Process

The heatstroke prevention process by the heatstroke prevention system 100 will be described with reference to FIG. 17. First, in the wearable terminal 30, the information acquisition unit 15 of the controller 10 acquires the information on the auricle temperature as the sensor information measured by the auricle thermo-hygro sensor 11, at a preset timing.

The information acquisition unit 15 transmits the acquired information on the auricle temperature to the communication circuitry of the wearable terminal 30, and causes the storage unit 18 to store the information on the auricle temperature. The communication circuitry of the wearable terminal 30 sends the received information on the auricle temperature to the server 40 and the management device 50 through the network 200.

Next, in the server 40, the communication circuitry of the server 40 receives the information on the auricle temperature from the wearable terminal 30 through the network 200. The communication circuitry of the server 40 transmits the received information on the auricle temperature to the arithmetic device of the server 40, and causes the storage unit of the server 40 to store the information on the auricle temperature.

The arithmetic device of the server 40 reads the threshold set for the auricle temperature, from the storage unit of the server 40, compares the information on the auricle temperature transmitted from the communication circuitry of the server 40 and the threshold read from the storage unit of the server 40, and determines whether the auricle temperature is higher than the threshold. In the case where the auricle temperature is higher than the threshold, the arithmetic device of the server 40 determines that there is the possibility that the biological body develops the heatstroke.

On the other hand, in the case where the auricle temperature is lower than or equal to the threshold, the arithmetic device of the server 40 determines that there is a low possibility that the biological body develops the heatstroke. Then, the arithmetic device of the server 40 transmits the determination result to the communication circuitry of the server 40, and causes the storage unit of the server 40 to store the determination result.

The communication circuitry of the server 40 transmits the received information providing the determination result about the heatstroke, to the wearable terminal 30, the management device 50, and the display 60 through the network 200.

In the wearable terminal 30, the communication circuitry of the wearable terminal 30 receives the information providing the determination result about the heatstroke, from the server 40 through the network 200. The communication circuitry of the wearable terminal 30 transmits the received information providing the determination result, to the operation control unit 17, and causes the storage unit 18 to store the determination result.

In the case where the received determination result indicates that there is a high possibility of the development of the heatstroke, the operation control unit 17 controls the notification means 14, and causes the notification means 14 to give the notice that there is the possibility of the development of the heatstroke. Thereby, the notice of the possibility of the development of the heatstroke is given to the user. At this time, the operation control unit 17 controls the cooling means 21 such that the cooling means 21 cools the periphery of the auricle of the biological body. Thereby, the brain temperature is decreased, and the development of the heatstroke is inhibited.

Meanwhile, in the management device 50, the communication circuitry of the management device 50 receives the information on the auricle temperature sent from the wearable terminal 30. The communication circuitry of the management device 50 receives the information providing the determination result about the heatstroke and sent from the server 40. The communication circuitry of the management device 50 transmits the received information to the controller of the management device 50.

The controller of the management device 50 causes the storage unit of the management device 50 to store the received information, and causes the display means of the management device 50 to display necessary information. For example, in the case where the received information on the determination result indicates that there is the possibility that the biological body develops the heatstroke, the controller of the management device 50 causes the display means of the management device 50 to display information on the biological body corresponding to the received information.

The display 60 receives the information providing the determination result about the heatstroke from the server 40 through the network 200, and displays the information on the display.

Thereby, for the biological body displayed on the display means of the management device 50, the manager inputs, for example, an operation to give the biological body a break, using the operation unit of the management device 50. In this case, the controller of the management device 50 issues a command on the basis of the input operation, and transmits the command to the communication circuitry of the management device 50.

The communication circuitry of the management device 50 sends the received command to the wearable terminal 30 through the network 200. Thereby, the wearable terminal 30 having received the command is configured to give a notice corresponding to the command.

As described above, in the heatstroke prevention system 100 according to Embodiment 3, it is possible to determine the possibility of the development of the heatstroke easily and surely, and to prevent the heatstroke, similarly to Embodiment 1. Further, in Embodiment 3, as the condition of the biological body is managed by the management device 50, it is possible to cause the biological body to take an appropriate measure for inhibiting the development of the heatstroke, by sending the command depending on the condition of the biological body.

In Embodiment 3, the determination of the possibility of the development of the heatstroke is performed by the server 40, but without being limited to this manner, may be performed by the wearable terminal 30. Specifically, the wearable terminal 30 measures the auricle temperature using the auricle thermo-hygro sensor 11, compares the measured auricle temperature and the threshold, and determines the possibility of the development of the heatstroke. Then, the wearable terminal 30 sends information providing the determination result to the server 40 through the network 200.

The server 40 receives the information providing the determination result about the possibility of the development of the heatstroke, and sends the received information providing the determination result, to the wearable terminal 30, the management device 50, and the display 60, through the network 200. Subsequently, the same processes as those in the above example are performed. Thus, even when the determination of the possibility of the development of the heatstroke is performed by the wearable terminal 30, it is possible to determine the possibility of the development of the heatstroke easily and surely, and to prevent the heatstroke.

### LIST OF REFERENCE SIGNS

- 1: heatstroke prevention device
- 10: controller
- 11: auricle thermo-hygro sensor
- 12: environment thermo-hygro sensor
- 13: acceleration sensor
- 14: notification means
- 15: information acquisition unit
- 16: comparison unit
- 17: operation control unit
- 18: storage unit
- 21: cooling means
- 30: wearable terminal
- 40: server
- 50: management device
- 60: display
- 71: processing circuit
- 72: processor
- 73: memory
- 100: heatstroke prevention system
- 200: network

## Claims

1. A heatstroke prevention device comprising:
- an auricle temperature sensor configured to measure an auricle temperature that is a temperature of an auricle of a biological body;
- a controller configured to determine a possibility of development of heatstroke of the biological body on the basis of the auricle temperature; and
- a notification means configured to give a notice that there is the possibility of the development of the heatstroke on the basis of a determination result of the controller,
the controller including
- a comparison unit configured to compare the measured auricle temperature and a preset threshold of the auricle temperature for the heatstroke, and to determine whether there is the possibility of the development of the heatstroke on the basis of a comparison result, and
- an operation control unit configured to control operation of the notification means on the basis of a determination result of the comparison unit.

2. A heatstroke prevention device comprising:
- an auricle temperature sensor configured to measure an auricle temperature that is a temperature of an auricle of a biological body;
- a controller configured to determine a possibility of development of heatstroke of the biological body on the basis of the auricle temperature; and
- a communication circuitry configured to send a determination result of the controller to a server,
the controller including
- a comparison unit configured to compare the measured auricle temperature and a preset threshold of the auricle temperature for the heatstroke, and to determine whether there is the possibility of the development of the heatstroke on the basis of a comparison result.

3. The heatstroke prevention device of claim 1 or 2,
wherein the auricle temperature sensor measures a temperature of an auricular lobule of the biological body.

4. The heatstroke prevention device of any one of claims 1 to 3,
wherein, when the auricle temperature is higher than the preset threshold, the comparison unit determines that there is the possibility of the development of the heatstroke.

5. The heatstroke prevention device of any one of claims 1 to 4,
further comprising a cooling means configured to cool a periphery of the auricle, wherein,
when the controller determines that there is the possibility of the development of the heatstroke, the controller is configured to control the cooling means such that the cooling means cools the periphery of the auricle.

6. The heatstroke prevention device of any one of claims 1 to 5,
wherein the preset threshold is set on the basis of a parameter including any of a surrounding environment temperature, an auricular lobule temperature, a humidity, an atmospheric pressure, a labor time, a working time, an active mass, a sex and an age.

7. A heatstroke prevention system comprising:
- a wearable terminal;
- a server configured to perform communication with the wearable terminal; and a management device configured to perform communication with the server, the wearable terminal including
- an auricle temperature sensor configured to measure an auricle temperature that is a temperature of an auricle of a biological body, and
- a notification means configured to give a notice that there is a possibility of development of heatstroke of the biological body,
the server including an arithmetic device configured to compare the auricle temperature measured by the wearable terminal and a preset threshold of the auricle temperature, and to determine whether there is the possibility of the development of the heatstroke on the basis of a comparison result,
the management device including
- a storage unit configured to store a determination result of the server, and
- a display means configured to display the determination result.

8. A heatstroke prevention system comprising:
- a server configured to perform communication with a wearable terminal; and
- a management device configured to perform communication with the server,
the server including an arithmetic device configured to compare an auricle temperature and a preset threshold of the auricle temperature, and to determine whether there is a possibility of development of heatstroke on the basis of a comparison result, the auricle temperature being a temperature of an auricle of a biological body and being measured by the wearable terminal,
the management device including
- a storage unit configured to store a determination result of the server, and
- a display means configured to display the determination result.

9. A heatstroke prevention method comprising:
- measuring an auricle temperature that is a temperature of an auricle of a biological body;
- comparing the measured auricle temperature and a preset threshold of the auricle temperature for heatstroke; and
- controlling operation of a notification means on the basis of a result of the comparing.

10. A program causing a computer of a wearable terminal to be used as a comparison unit and an operation control unit, the wearable terminal including an auricle temperature sensor configured to measure an auricle temperature that is a temperature of an auricle of a biological body, and a notification means configured to give a notice that there is a possibility of development of heatstroke,
- the comparison unit comparing the measured auricle temperature and a preset threshold of the auricle temperature for the heatstroke, and determining whether there is the possibility of the development of the heatstroke on the basis of a comparison result,
- the operation control unit controlling operation of the notification means on the basis of a determination result of the comparison unit.
